Europäisches Patentamt

European Patent Office

Office européen des brevets

(19)

(11) Publication number: **0 270 668 B1**

## EUROPEAN PATENT SPECIFICATION

(12)

(45) Date of publication of patent specification: **02.09.92**

(51) Int. Cl.[5]: **A61M 5/168**

(21) Application number: **87904996.3**

(22) Date of filing: **28.05.87**

(86) International application number:
**PCT/US87/01207**

(87) International publication number:
**WO 87/07161 (03.12.87 87/27)**

(54) DROP DETECTION HOUSING WITH POSITIVE TACTILE SIGNALING.

(30) Priority: **28.05.86 US 868258**

(43) Date of publication of application:
**15.06.88 Bulletin  88/24**

(45) Publication of the grant of the patent:
**02.09.92 Bulletin  92/36**

(84) Designated Contracting States:
**BE DE FR GB SE**

(56) References cited:
**EP-A- 0 229 354    DE-U- 8 335 578**
**FR-A- 2 546 068    US-A- 4 037 597**
**US-A- 4 321 461    US-A- 4 346 606**
**US-A- 4 397 648    US-A- 4 533 350**
**US-A- 4 557 725    US-A- 4 668 216**

**GRUNDIG TECHNISCHE INFORMATIONEN,
vol. 28, no. 3, 1981, pages 119-128, Regens-
burg, DE; F. MEIERHÖFER: "Das Bedienteil
und die Uhr des GRUNDIG-Videorecorders
Video 2X4 super"**

**US, A, DES 279602**

**US, A, DES 273419**

(73) Proprietor: **DEKA PRODUCTS LIMITED PART-
NERSHIP
340 Commercial Street
Manchester, NH 03101(US)**

(72) Inventor: **Kamen, Dean L.
44 Gage Road
Bedford, New Hampshire 03102(US)**

(74) Representative: **MacGregor, Gordon et al
ERIC POTTER & CLARKSON St. Mary's Court
St. Mary's Gate
Nottingham, NG1 1LE(GB)**

Note: Within nine months from the publication of the mention of the grant of the European patent, any person
may give notice to the European Patent Office of opposition to the European patent granted. Notice of opposition
shall be filed in a written reasoned statement. It shall not be deemed to have been filed until the opposition fee
has been paid (Art. 99(1) European patent convention).

EP 0 270 668 B1

## Description

### Technical Field

The present invention relates to a medical infusion apparatus according to the first part of claim 1. Such a medical infusion apparatus is disclosed by the US-A-4 321 461.

### Background of the Invention

Medical infusion controllers now provide microprocessor control of intravenous fluid delivery rates. These devices include sensors that monitor fluid flow rate and then adjust that rate accordingly. Despite the automatic nature of these devices, they typically must be manually set by the operator to indicate to the microprocessor whether the particular infusion set in use contains a 10 cm$^3$ or a 60 cm$^3$ cannula. Thus, there is a potential for serious human error. EP-A-022 9354 discloses a device having a protruding spring-loaded retractable pin switch on the controller. 10-cm$^3$ and 60-cm$^3$ cannula drip chamber are distinguished by the presence or absence of an aperture to receive the pin. If the aperture is absent, the pin is urged into the retracted position. If the aperture is present, the pin fits into the aperture, and thus does not retract. The two pin positions are associated with two different modes of operation. It can be seen that there is room for error, inasmuch as misalignment or malfunction of the pin can cause incorrect data to be signalled to the controller, by either retracting even in the presence of an aperture, or by failing to retract even in the absence of an aperture.

### Disclosure of Invention

The present invention provides a system for automatically switching the controller between the 10 cm$^3$ cannula setting and the 60 cm$^3$ cannula setting. Rather than the retractable pin switch in the prior art, the present invention provides a plurality of pressure activated switches on the controller. These switches are configured in such a way that the controller will be set to a desired mode of operation when one and only one switch is activated. When more or less than one switch is activated, the controller will deliver an error message to the user. Drip chambers with different sized cannulae are provided with nubs in one of a variety of configurations designed to set the controller's mode of operation to correspond with that particular drip chamber's cannula size. When the drip chamber is placed in the controller, the nub comes into contact with the appropriate switch and the controller is thereby set to the appropriate mode of operation.

Thus, the present system, which conveys a positive signal to the control based on the presence of a nub in a particular location, avoids the aforementioned potential for error in the prior art device. As discussed above, the controller will send an error message and/or enter into an alarm mode unless an affirmatively correct signal is received. Thus, unlike the prior art device, the present device will not operate if, for example, a drip chamber from another manufacturer is placed in the controller, or if some of the contact sites are misaligned.

The characterising features of the invention are set out in the characterising part of Claim 1.

### Brief Description of the Drawings

Fig. 1 shows a side elevational view of one possible embodiment of a drip chamber assembly according to the present invention.

Fig. 2 shows a elevational view from above of the flange element of the drip chamber assembly shown in Fig. 1.

Fig. 3 shows a perspective view from above of one possible medical infusion controller for use with the drip chamber assembly shown in Fig. 1.

Fig. 4 shows a perspective view from above of the drip chamber of Fig. 1 placed in the medical infusion controller of Fig. 3.

### Description of Specific Embodiments

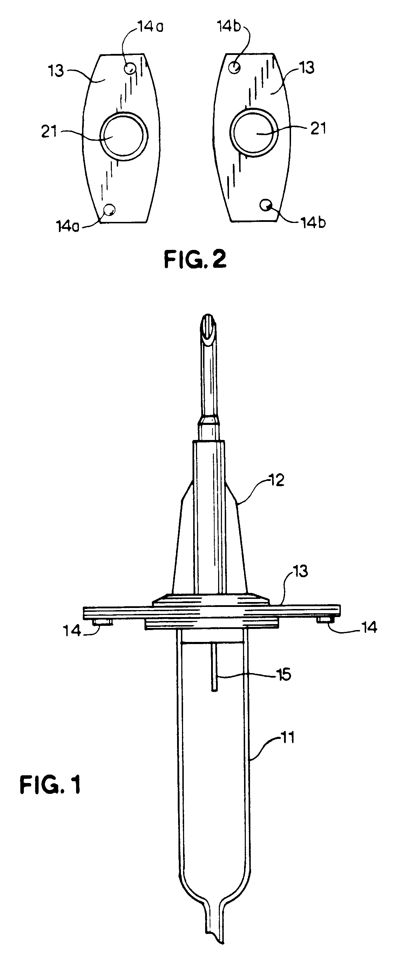

Fig. 1 shows one possible embodiment of the present invention. The drip chamber 11 with cannula 15 and spike element 12 is provided with a flange element 13. The flange element is provided with a pair of nubs 14 that are disposed in such a way that whichever side of the flange is presented to a medical infusion controller, the nub on that side of the flange is located in the same relative position.

This will be better understood in Fig. 2, which shows two possible configurations of nubs on the flange element. It will be seen that the nubs are radially symmetric to each other around the center of the drip chamber area 21. Thus, regardless of which end of the flange is up, nub 14a will always be in the righthand corner and nub 14b will always be in the lefthand corner.

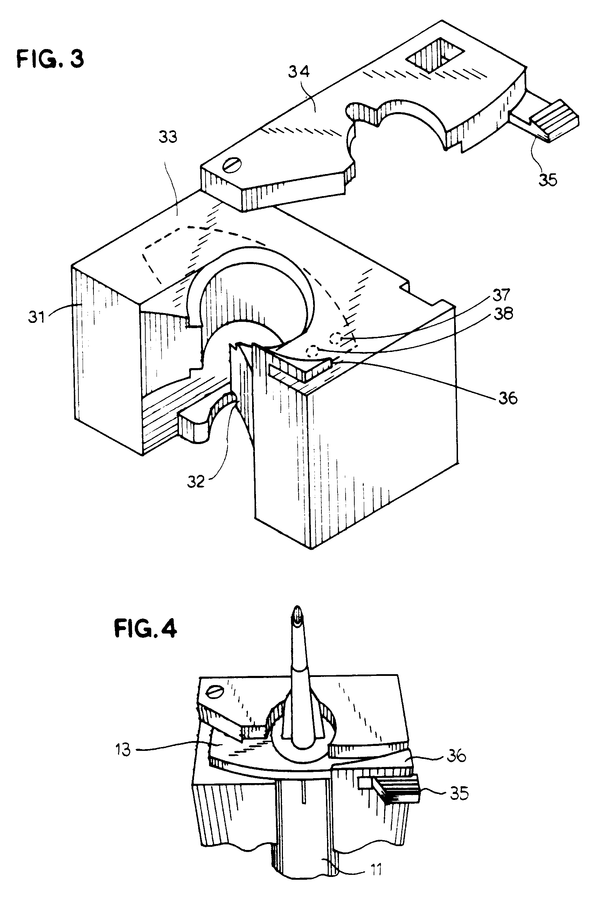

Fig. 3 shows a possible medical infusion controller for use in conjunction with the drip chamber shown in Fig. 1. The body 31 of the controller is provided with an aperture 32 for receiving the drip chamber. Surface 33 of the controller receives the flange element. The flange element is held in place by hinged retaining element 33, the end of which 35 locks into mating element 36. The mode of operation of the controller is set by the actuation of either switch 37 or 38. Although two switches are shown, the invention would apply to any number of switches greater than one.

Using means well known in the art, the controller is designed to enter one of various modes of operation upon the activation of exactly one switch. When more or less than one switch is activated, the controller will deliver an error message to the user. It would be possible as well for the controller to enter an alarm mode.

In the present embodiment, it is contemplated to use the switching system to switch the controller to either a mode of operation appropriate to a 10-cm$^3$ cannula or to a mode of operation appropriate to a 60-cm$^3$ cannula. However, it will be clear that the inventive concept is not limited to switching to these two particular modes of operation. The switching system could be modified to include a greater number of nubs in a variety of configurations, thus enabling foolproof switching to one of a potentially large number of mode settings. Further, the modes of operation governed by the switching system are not limited to cannula size. Conceivably, they could include content of the infusion bag, maximum flow rate, model of controller that is expected, characteristics of flow desired for a given class of patient, etc.

Because of the automatic setting of the controller, and the error message delivered upon presentation of an inappropriate nub number or configuration, the present system provides a safety device. It will be readily apparent that the system guards against undesired settings resulting either from human error or from those errors resulting from the use of retractable pin-type switches discussed earlier.

Fig. 4 shows the drip chamber of Fig. 1 placed in the controller of Fig. 3. The nub on the side of the flange element over the switches is urged onto the corresponding switch by retaining element 34. As can be seen, it makes no difference which side of the flange element is over the switches. As discussed above, because of the symmetry of the flange element and the symmetrical configuration of the nubs on the flange element, either side of the flange element causes the activation of the same switch, and thus produces the same setting of the controller's mode of operation.

## Claims

1. Medical infusion apparatus including a plurality of drip chambers (11), each with a flange (13) affixed thereto, the drip chambers having different cannula sizes and an infusion controller (31) having switching means for switching the controller between a plurality of modes of operation,

   characterised in that:-

   each drip chamber (11) has a preset number of tactile signalling nubs (14), comprising at least one nub, affixed to the flange (13) in one of a plurality of predetermined alternative positions, each said position indicating the cannula size of the drip chamber,

   and the switching means is located in the controller so as to be responsive to the presence of said preset number of nubs (14) in any one of said predetermined positions on the flange (13) when a drip chamber (11) is engaged with the controller, so as to provide a mode of operation corresponding to the size of cannula in the drip chamber, the switching means sending an error message when any number of nubs other than said preset number of nubs is detected.

2. Apparatus according to claim 1, wherein the flange (13) includes a pair of symmetrically disposed wing members and each of the wing members includes a nub (14) so disposed that the desired mode of operation is signaled independent of which of the two wings is used for signaling.

3. Apparatus according to claim 1 or 2, wherein the switching means (37,38) comprises a plurality of switching sensors (37,38) each positioned to be individually responsive to the presence of a nub (14) in a predetermined position on the flange (13) affixed to a drip chamber (11).

4. Apparatus according to claim 3, wherein the controller includes a body (31) provided with an aperture (32) for receiving the drip chamber (11) and retaining means (33) for holding the flange (13) of the drip chamber in place, whereby a said nub (14) on the flange (13) is located for actuation of the switching means (37,38).

## Patentansprüche

1. Medizinische Infusionseinrichtung, umfassend eine Mehrzahl von Tropfkammern (11), jede mit einem daran befestigten Flansch (13), wobei die Tropfkammern unterschiedliche Kanülgrößen haben und eine Infusionssteuer- bzw. -regeleinrichtung (31), die eine Schalteinrichtung zum Umschalten der Steuer- bzw. -regeleinrichtung zwischen einer Mehrzahl von Betriebsweisen hat,

   dadurch **gekennzeichnet**, daß

   jede Tropfkammer (11) eine festgesetzte Anzahl von tastbaren Signalisierungsnoppen (14) hat, umfassend wenigstens eine Noppe, die an dem Flansch (13) in einer aus einer Mehrzahl von vorbestimmten alternativen Positionen befestigt sind, wobei jede Position die Kanülgröße der Tropfkammer angibt,

und sich die Schalteinrichtung so in der Steuer- bzw. -regeleinrichtung befindet, daß sie auf das Vorhandensein der festgesetzten Anzahl von Noppen (14) in irgendeiner der vorbestimmten Positionen auf dem Flansch (13) anspricht, wenn eine Tropfkammer (11) mit der Steuer- bzw. -regeleinrichtung in Eingriff gebracht wird, so daß eine Betriebsweise vorgesehen wird, welche der Größe der Kanüle in der Tropfkammer entspricht, wobei die Schalteinrichtung eine Fehlerbotschaft sendet, wenn irgendeine andere Anzahl von Noppen als die festgesetzte Anzahl von Noppen detektiert wird.

2. Einrichtung nach Anspruch 1, worin der Flansch (13) ein Paar von symmetrisch angeordneten Flügelteilen umfaßt und jedes der Flügelteile eine Noppe (14) aufweist, die so angeordnet ist, daß die gewünschte Betriebsart signalisiert wird, unabhängig davon, welcher der beiden Flügel für die Signalisierung verwendet wird.

3. Einrichtung nach Anspruch 1 oder 2, worin die Schalteinrichtung (37, 38) eine Mehrzahl von Schaltsensoren (37, 38) umfaßt, von denen jeder so positioniert ist, daß er individuell auf das Vorhandensein einer Noppe (14) in einer vorbestimmten Position auf dem Flansch (13), der an einer Tropfkammer (11) befestigt ist, anspricht.

4. Einrichtung nach Anspruch 3, worin die Steuer- bzw. -regeleinrichtung einen Körper (31) aufweist, der mit einer Öffnung (32) zum Aufnehmen der Tropfkammer (11) und einer Halteeinrichtung (33) zum Halten des Flanschs (13) der Tropfkammer an Ort und Stelle versehen ist, wodurch eine Noppe (14) auf dem Flansch (13) zur Betätigung der Schalteinrichtung (37, 38) lokalisiert wird.

**Revendications**

1. Dispositif de perfusion médicale incluant une pluralité de chambres de goutte à goutte (11), chacune avec une bride (13) fixée sur elle, les chambres de goutte à goutte ayant différentes dimensions de canules et un contrôleur de perfusion (31) ayant des moyens de commutation pour conmuter le contrôleur entre une pluralité de modes de fonctionnement, caractérisé en ce que :

chaque chambre de goutte à goutte (11) comporte un nombre prédéterminé de boutons de signalisation tactile (14), comprenant au moins un bouton, fixé à la bride (13) dans l'une des positions d'une pluralité d'autres positions prédéterminées, chaque dite position indiquant la dimension de la canule de la chambre de goutte à goutte,

et en ce que le moyen de commutation est situé dans le contrôleur de façon à être sensible à la présence dudit nombre prédéterminé de boutons (14) dans l'une quelconque desdites positions prédéterminées sur la bride (13) quand une chambre de goutte à goutte (11) est engagée avec le contrôleur, de façon à fournir un mode de fonctionnement correspondant à la dimension d'une cannule dans la chambre de goutte à goutte, le moyen de commutation envoyant un message d'erreur lorsque n'importe quel nombre de boutons autre que ledit nombre prédéterminé de boutons est détecté.

2. Dispositif selon la revendication 1, dans lequel la bride (13) inclut une paire de surfaces d'appui disposée symétriquement et chacune de ces surfaces inclut un bouton (14) disposé de sorte que le mode de fonctionnement souhaité est signalé indépendamment de celle des deux surfaces d'appui qui est utilisée pour la signalisation.

3. Dispositif selon la revendication 1 ou 2, dans lequel le moyen de commutation (37, 38) comporte une pluralité de capteurs de commutation (37, 38) positionné chacun pour être sensible individuellement à la présence d'un bouton (14) dans une position prédéterminée sur la bride (13) fixée à la chambre de goutte à goutte (11).

4. Dispositif selon la revendication 3, dans lequel le contrôleur inclut un corps (31) muni d'une ouverture (32) pour recevoir la chambre de goutte à goutte (11) et des moyens de retenue (33) pour maintenir en place la bride (13) de la chambre de goutte à goutte, de façon qu'un dit bouton (14) sur la bride (13) soit placé pour actionner les moyens de commutation (37, 38).

FIG. 2

FIG. 1

# FIG. 3

# FIG. 4